## Europäisches Patentamt
## European Patent Office
## Office européen des brevets

(19)

(11) Veröffentlichungsnummer: **0 088 964**

**B1**

(12) # EUROPÄISCHE PATENTSCHRIFT

(45) Veröffentlichungstag der Patentschrift:
02.09.87

(21) Anmeldenummer: 83102132.4

(22) Anmeldetag: 04.03.83

(51) Int. Cl.⁴: **B 01 J 31/06,** B 01 J 20/26,
B 01 J 39/20, C 12 N 11/08,
C 12 P 1/00, C 07 B 41/00,
C 07 B 61/00

(54) Verfahren zur Herstellung von unlöslichen, nur wenig quellbaren Polymerisaten von basischen Vinylheterocyclen und deren Verwendung.

(30) Priorität: 13.03.82 DE 3209224

(43) Veröffentlichungstag der Anmeldung:
21.09.83 Patentblatt 83/38

(45) Bekanntmachung des Hinweises auf die Patenterteilung:
02.09.87 Patentblatt 87/36

(84) Benannte Vertragsstaaten:
AT BE CH DE FR GB IT LI NL SE

(56) Entgegenhaltungen:
FR-A-2 206 335
FR-A-2 281 383

Die Akte enthält technische Angaben, die nach
dem Eingang der Anmeldung eingereicht wurden
und die nicht in dieser Patentschrift enthalten sind.

(73) Patentinhaber: BASF Aktiengesellschaft, Carl-Bosch- Strasse 38, D-6700 Ludwigshafen (DE)

(72) Erfinder: Denzinger, Walter, Wormser Landstrasse 65, D-6720 Speyer (DE)
Erfinder: Goertz, Hans- Helmut, Dr., An der Froschlache 23, D-6700 Ludwigshafen (DE)
Erfinder: Sanner, Axel, Dr., Lorscher Ring 2C, D-6710 Frankenthal (DE)
Erfinder: Hartmann, Heinrich, Dr., Weinheimer Strasse 46, D-6703 Limburgerhof (DE)

LIBER, STOCKHOLM 1987

## Beschreibung

Bekanntlich sind Homopolymere von N-Vinylimidazolen und Vinylpyridinen sowie deren Copolymere mit N-Vinyllactamen in Wasser und zahlreichen organischen Lösungsmitteln leicht löslich. Es ist auch bekannt, daß man unlösliche, aber quellbare Polymerisate von Vinylimidazolen bzw. Vinylpyridinen herstellen kann, indem man die Vinylheterocyclen in üblicher Art und Weise in Gegenwart mehrfunktioneller Monomerer (mit zwei oder mehr copolymerisierbaren Doppelbindungen), die vernetzend wirken, und gegebenenfalls einfach ungesättigter Comonomerer unter Zusatz von Radikalbildnern als Starter (oder Initiator genannt) polymerisiert. Als Vernetzer kommen beispielsweise in Frage Divinylester von Dicarbonsäuren wie Bernsteinsäure und Adipinsäure, Diacrylate von mehrwertigen Alkoholen wie Ethylenglykol und Butandiol, Allylether von mehrwertigen Alkoholen wie Pentaerythrittriallylether. Derartige Vernetzer sind nicht ganz einfach herzustellen und daher im Vergleich zu einfach ungesättigten Monomeren relativ teuer. Selbst bei Einsatz größerer Mengen des mehrfunktionellen Monomeren ist die Quellbarkeit der Polymeren in Wasser relativ hoch, so daß bei der Polymerisation in wäßriger Lösung ein Gel entsteht.

So lehrt die US-A- 2 878 183 die Herstellung von sehr stark vernetzten und daher unlöslichen, aber trotzdem stark quellbaren Polyvinylimidazolen durch radikalische Polymerisation von N-Vinylimidazolen in Gegenwart großer Mengen mehrfunktioneller Monomerer, wobei die Polymerisation in Wasser durchgeführt wird und die Polymeren als Gele anfallen. Solche stark quellbaren Gele haben sowohl bei ihrer Herstellung wie bei der allgemeinen Handhabung und bei der Anwendung als Ionenaustauscher oder als Träger für Reagentien wie Enzyme und dgl. schwerwiegende Nachteile: Sie verstopfen bei der Herstellung das Gefäß, können weder gerührt noch ausgeschüttet werden, und beim Trocknen müssen große Lösungsmittelmengen verdampft werden. Zum Gebrauch müssen sie vorgequollen werden und sind in diesem Zustand nicht schüttfähig; eine damit gefüllte Kolonne neigt zum Verstopfen.

Die DE-AS 19 29 501 beschreibt die Herstellung von Copolymeren aus N-Vinyllactamen mit geringen Mengen an N-Vinylimidazolen durch radikalische Polymerisation in Gegenwart von Vernetzern, wobei die Polymerisation in 10 bis 20 %igen wäßrigen Salzlösungen durchgeführt wird. Die Polymeren fallen dann in Form von Granulaten mit 1 bis 7 mm Durchmesser an. Nachteile dieses Verfahrens bestehen u.a. darin, daß die Isolierung der Polymeren in reiner Form aus der Salzlösung sehr aufwendig ist, und daß das Polymere in grober, nicht poröser Form und mit relativ geringer Oberfläche anfällt.

Eine Methode zur Herstellung von Poly-N-vinylimidazolen beschreibt die DE-OS 23 24 204, wobei die Polymerisation in einem organischen Lösungsmittel erfolgt und es sehr aufwendig ist, die Polymeren in lösungsmittelfreier Form zu isolieren.

Die DE-OS 25 06 085 lehrt die Polymerisation von Vinylimidazolen ohne Verwendung von Lösungsmitteln durch Photoinitiierung. Da die Polymerisate hierbei als Folien erhalten werden, ist ihr Einsatz stark eingeschränkt. Von Schwächen bei der Herstellung abgesehen, sind diese Polymerisate aufgrund ihrer relativ starken Quellbarkeit auch nur in begrenztem Maße als Ionenaustauscher oder Träger für Proteine geeignet.

Ein Verfahren zur Erzeugung relativ schwach quellbarer Polymerisate, auch ohne Zusatz größerer Vernetzermengen ist in der Literatur u.a. als Popcorn-Polymerisation (R.F. Kauffmann und J. W. Breitenbach, Angew. Makromol. Ch. 45 (1975), 167 bis 175: N-Vinylpyrrolidon und Popcorn-Polymere; J.W. Breitenbach, R.F. Kauffmann und G. Zwilling, Makromol. Ch. 177 (1976), 2787 bis 2792: Acrylsäure-Popcorn-Polymere) oder im Englischen auch als Proliferous Polymerization (W. Breitenbach, Encycl. of Polymer Sci. and Technol. Vol. 11, 587 bis 597) bekannt. Diese Polymerisationsart, in der Technik als Störquelle bekannt, führt in der Regel zu technisch unbrauchbaren, unregelmäßig grobteiligen Produkten. Sie ist für basische Vinylheterozyklen bisher nicht bekannt.

Der Erfindung lag die Aufgabe zugrunde, in möglichst einfacher Weise und unter Einsatz von möglichst geringen Mengen an (teuren) Vernetzern mit zwei oder mehr copolymerisierbaren Doppelbindungen porös körnige, in Wasser schwach quellbare, nicht gelbildende basische Polymere herzustellen, die als Ionenaustauscher und als Adsorberharze (Träger), insbesondere für Proteine (vor allem Enzyme), geeignet sind.

Die Lösung dieser Aufgabe wurde in dem Verfahren nach den Herstellansprüchen gefunden.

Die erfindungsgemäß erhältlichen Polymeren unterscheiden sich von den "normalen" d.h. mit Hilfe von üblichen Mengen an Radikalbildnern als Startern oder durch Photoinitiierung erhaltenen Polymeren nicht nur dadurch, daß sie bei gleicher Vernetzerkonzentration wesentlich weniger in Wasser quellen, so daß die durch Quellung aufgenommene Wassermenge weniger als die Hälfte, in der Regel ein Drittel bis ein Siebentel oder noch weniger beträgt, sondern auch im gesamten Polymerisationsablauf und vor allem in ihrer optischen (makro- und mikroskopischen) Erscheinung: eine "normale" Polymerisation ohne Lösungsmittel ergibt einen festen Block, und in wäßriger Lösung im Falle der Wasserlöslichkeit von Monomer und Polymer (wie im vorliegenden Fall) ein Gel, während hier in beiden Fällen eine poröse, körnige Masse mit einem Korndurchmesser im Bereich von etwa 10 bis 500 μm entsteht. Wird ohne Rühren polymerisiert, so verbacken die Körner zu einem porösen Kuchen, der sich leicht zerkrümeln läßt. Wird während der Polymerisation gerührt, was stets vorzuziehen ist, so agglomerieren die Körner lose zu unregelmäßig geformten Krümeln, deren durchschnittliche Größe von der Rührintensität abhängt und meist in der Größenordnung von 0,1 bis 5 mm Durchmesser liegt.

Die Polymerisation des Monomeren/Vernetzer-Gemisches beginnt, sobald der Luftsauerstoff vollständig entfernt ist, spontan selbst bei Raumtemperatur und sogar ohne daß die Polymerisationsinhibitoren, beispielsweise Hydrochinon, t-Butylbrenzkatechin oder Phenothiazin, die die Monomeren normalweise zur Erhöhung ihrer Lagerfähigkeit enthalten, entfernt worden wären. Molekularer Sauerstoff erscheint als der einzige Inhibitor, der nicht nur die normale radikalische, sondern auch die erfindungsgemäße Polymerisation inhibiert.

Aus dem deutschen Patent 24 37 629 ist ein Verfahren zur Herstellung von Klärungsmitteln für vegetabilische Getränke bekannt, demzufolge Mischungen von N-Vinyllactamen mit geringen Mengen eines zweifach ungesättigten cyclischen Säureamids als Vernetzer in Gegenwart bestimmter Schwefelverbindungen in verdünnter wäßriger Lösung nach der gleichen Polymerisationsmethode wie gemäß der vorliegenden Erfindung polymerisiert werden. Die so erhältlichen Polymerisate adsorbieren besonders gut Gerbstoffe, jedoch nich Proteine, sind also nich als Träger für Enzyme außerdem nicht als Ionenuastauscherharze geeignet. Das gleiche gilt für nach DE-C 22 55 263 in ähnlicher Weise, jedoch ohne Scnwefelverbindungen erhältliche Pyrrolidon-Polymerisate.

Es ist das Verdienst der vorliegenden Erfindung, diese bei anderen Monomeren an sich bekannte, in der Regel aber nur unter negativen Aspekten betrachtete, weil als störend empfundene Polymerisationsart zum ersten Mal gezielt auf basische Vinylheterocyclen angewandt und dabei überraschend ein technisch fortschrittliches Ergebnis erreicht zu haben. Es gelingt erfindungsgemäß auf einfache Weise, die oben beschriebene Aufgabe zu lösen, d. h. in Wasser schwach quellbare und daher nicht gelbildende basische Polymere (und zwar gleich mit der erforderlichen großen Oberfläche) herzustellen, die als Ionenaustauscher und Adsorberharze hervorragend geeignet sind und ein breites Anwendungsspektrum haben.

Unter basischen Vinylheterocyclen sind hier gesättigte und aromatisch ungesattigte Heterocyclen mit einer Vinylgruppe und mindestens einem basischen tertiären Ring-Stickstoffatom mit einem pKa über 4, vorzugsweise im Bereich von 5 bis 8, zu verstehen. Außer der Vinylgruppe kann der Ring auch Alkylgruppen mit 1 bis 4 Kohlenstoffatomen, Phenyl-, Benzyl-Gruppen oder auch einen anellierten zweiten Ring tragen. Als Beispiele seien genannt: N-Vinylimidazol sowie Derivate wie 2-Methyl-1-vinylimidazol, 4-Methyl-1-vinylimidazol, 5-Methyl-l-vinylimidazol, 2-Ethyl-1-vinylimidazol, 2-Propyl-1-vinylimidazol, 2-Isopropyl-1-vinylimidazol, 2-Phenyl-1-vinylimidazol, 1-Vinyl-4,5-benzimidazol, wobei N-Vinylimidazol und 2-Methyl-1-vinylimidazol besonders bevorzugt sind. Weiterhin können beispielsweise eingesetzt werden: 2-Vinylpyridin, 4-Vinylpyridin sowie 5-Methyl-2-vinylpyridin. Selbstverständlich können auch Gemische von basischen Vinylheterocyclen untereinander eingesetzt werden.

Das Vernetzungsmittel wird in Mengen von 0,1 bis 10, vorzugsweise 1 bis 4 %, bezogen auf das Gesamt-Monomerengewicht, verwendet. Geeignete Vernetzer sind solche, die zwei oder mehr copolymerisierbare Gruppen im Molekül enthalten. Besonders geeignet sind Alkylenbisacrylamide wie Methylenbisacrylamid und N,N'-Bisacryloylethylendiamin, außerdem N,N'-Divinylethylenharnstoff, N,N'-Divinylpropylenharnstoff, Ethyliden-bis-3-(N-vinylpyrrolidon) sowie N,N'-Divinyldiimidazolyl(2,2') und 1,1'-Bis(3,3'-vinylbenzimidazolid-2-on)-1,4-butan. Andere brauchbare Vernetzungsmittel sind beispielsweise Alkylenglykoldi(meth)acrylate wie Ethylenglykoldi(meth)acrylat und Tetramethylenglykoldi(meth)acrylat, aromatische Divinylverbindungen wie Divinylbenzol und Divinyltoluol sowie Allylacrylat, Divinyldioxan, Pentaerythrit-triallylether und deren Gemische. Bei Polymerisation in Gegenwart von Wasser sind sie natürlich nur geeignet, soweit sie in der wäßrigen Monomermischung löslich sind.

Das gleiche gilt selbstverständlich für die Comonomeren, die in Mengen bis zu 30, vorzugsweise bis zu 20 Gew.%, bezogen auf die gesamte Monomermischung, einpolymerisiert werden können. Als solche kommen beispielsweise in Betracht Styrol, Acrylester, Vinylester, Acrylamid, vorzugsweise N-Vinyllactame wie 3-Methyl-N-vinylpyrrolidon, N-Vinylcaprolactam und insbesondere N-Vinylpyrrolidon.

Zur Durchführung der Polymerisation ohne Lösungsmittel wird das Monomerengemisch, bestehend aus basischem Vinylheterocyclus, dem vernetzenden Agens und gegebenenfalls N-Vinyllactam oder einem anderen Comonomeren durch Einleiten von Stickstoff inertisiert und anschließend auf 100 bis 200, vorzugsweise 150 bis 180°C erhitzt. Vorteilhaft ist es, wenn in die Mischung weiter ein schwacher Stickstoffstrom eingeleitet wird. Besonders vorteilhaft ist es, wenn der Ansatz durch Anlegen von Vakuum zum Sieden gebracht wird. Je nach Art der eingesetzten Monomeren und der gewählten Temperatur polymerisiert dann die Mischung innerhalb 1 bis 20 Stunden. Beispielsweise bilden sich bei der Polymerisation von 2-Methyl-1-vinylimidazol mit 2 % N,N'-Divinylethylenharnstoff bei 150°C unter Rühren mit einem kräftigen Rührwerk und einem Druck von 310 mbar nach 2,5 h die ersten Polymerisatteilchen, die langsam zunehmen, bis nach 10 h der Ansatz aus einem bräunlichen Pulver besteht. Nach Auswaschen mit Wasser und Trocknen wird das neue Polymere in Ausbeuten von über 90 % in Form eines groben Pulvers erhalten.

Eine bevorzugte Herstellungsart ist die Fällungspolymerisation in Wasser. Die Konzentration der Monomeren im Reaktionsansatz wird zweckmäßigerweise so gewählt, daß der Ansatz über die gesamte Reaktionsdauer hinweg gut rührbar bleibt. Bei zu wenig Wasser werden die Polymerisatkörner nämlich klebrig, so daß ein Rühren schwieriger wird als ganz ohne Wasser. Bei den üblichen Rührkesseln liegt die zweckmäßige Monomerenkonzentration, bezogen auf die wäßrige Mischung, bei ca. 5 bis 30, vorzugsweise 10 bis 20 Gew.%. Man kann sie bis auf 50 Gew.% steigern, wenn kräftige Rührwerke zur Verfügung stehen. Es kann auch zweckmäßig sein, die Polymerisation mit einer relativ konzentrierten Lösung zu beginnen und dann im Verlauf der Reaktion mit Wasser zu verdünnen. Die Polymerisation wird ggf. zweckmäßigerweise bei pH-Werten über 6 durchgeführt, um eine eventuell mögliche Verseifung der Comonomeren und/oder Vernetzer zu vermeiden. Die Einstellung des pH-Wertes kann durch Zugabe geringer Mengen Basen wie Natriumhydroxid oder Ammoniak oder der üblichen Puffersalze wie Soda, Natriumhydrogencarbonat oder Natriumphosphat erfolgen. Der Ausschluß von Sauerstoff läßt sich dabei dadurch erreichen, daß der Polymerisationsansatz am Sieden gehalten wird, und/oder wie erwähnt mit Hilfe eines Inertgases wie Stickstoff. Die Polymerisationstemperatur kann hier 20 bis 150° C betragen. Vorzugsweise arbeitet man bei 50 bis 100° C

Bisweilen kann es von Vorteil sein, zur völligen Entfernung von gelöstem Sauerstoff geringe Mengen - 0,1 bis 1 Gew.%, bezogen auf die Monomermischung, - eines Reduktionsmittels wie Natriumsulfit, Natriumpyrosulfit, Natriumdithionit, Ascorbinsäure und dergleichen zuzusetzen.

Bei einer besonders bevorzugten Ausführungsform der Fällungspolymerisation wird das wasserlösliche Comonomere (vorzugsweise ein N-Vinyllactam), ein Teil des Vernetzers, Wasser und gegebenenfalls ein Puffer und ein Reduktionsmittel in einem schwachen Stickstoffstrom erhitzt, bis sich die ersten Polymerisatteilchen zeigen. Dann wird eine vorher durch Einblasen von Stickstoff inertisierte Mischung aus dem Vinylheterozyklus und dem restlichen Vernetzer und gegebenenfalls Wasser als Verdünnungsmittel innerhalb 0,2 bis 2 Stunden zugegeben. Diese Arbeitsweise hat den Vorteil, daß sich die Polymerisationszeiten verkürzen.

Die Isolierung des mit etwa 90 bis 95 % der theoretischen Ausbeute anfallenden Polymeren aus der wäßrigen Suspension kann durch Filtrieren oder Zentrifugieren mit anschließendem Auswaschen mit Wasser und Trocknen in üblichen Trocknern wie Umluft- oder Vakuumtrockenschrank, Schaufeltrockner oder Stromtrockner erfolgen.

Aufgrund des Gehaltes an basischem Stickstoff sind die beanspruchten Polymerisate in nicht quaternierter Form als schwache, in quaternierter Form als starke Anionenaustauscher einsetzbar. Die vollständige oder teilweise Überführung in die quaternierte Form nach der Polymerisation mit üblichen Alkylierungsmitteln wie Methyljodid oder Benzylchlorid führt zu Austauschkapazitäten bis zu etwa 6m Val/g (Chlorid-Form). Abgesehen von ihrer Verwendbarkeit als Ionenaustauscher können die beanspruchten Polymerisate allgemein als Adsorberharze eingesetzt werden. Beispielsweise werden phenolische Substanzen wie Tannin adsorbiert. Ebenso können farbige Begleitsubstanzen aus Zuckerlösungen adsorptiv entfernt werden. Des weiteren adsorbieren diese Polymeren sehr gut Proteine, speziell Enzyme, welche in vielen Fällen auch nach der Adsorption einen beträchtlichen Teil ihrer Aktivität behalten. Sie können daher mit dem adsorbierten Enzym als heterogener Katalysator für die jeweilige Enzymreaktion eingesetzt werden. Besonders bevorzugt sind in diesem Fall die Enzyme Invertase, Glucoseisomerase, Amyloglucosidase, alpha- und beta-Amylase, Aminosäureacylase, Penicillinacylase und Hydantoinase. Ferner sind geeignet: Oxidoreduktasen - wie Alkoholdehydrogenase, Lactatdehydrogenase, Aminosäureoxidase, Peroxidase, Catecholoxidase, Monoaminooxidase, Lipoxygenase, Luciferase, Nitratreduktase, Nitritreduktase, Chlorperoxidase, Acetaldehyddehydrogenase, Aldehydoxigenase, Diaphorase, Cholesterinoxidase, Glutarthioreduktase, Hydroxysteroiddehydrogenase, Xanthinoxidase, Dopaminhydroxylase, Cytochromoxidase, Monoaminooxidase, Diacetylreduktase, Superoxiddismutase, Limonatdehydrogenase, Transferasen wie Polynucleotidphosphorylase, Dextransucrase, Phosphorylase, Carbamatkinase, Aminotransferase, Transaldolase, Methyltransferase, Pyruvatkinase, Carbamoyltransferase, Phosphofructokinase, Dextransynthetase, Hydrolasen wie Lipase, Esterase, Lactase, Lysozym, Cellulase, Urease, Trypsin, Chymotrypsin, Glutaminase, Asparaginase, Papain, Ficin, Pepsin, Leucinaminopeptidase, Carboxypeptidase A + B, Naringinase, Bromelain, Subtilisin, Phospholipase, Isoamylase, Cephalosporinamidase, Adenosindeaminase Penicillinase, Maltase, Dextranase, Desoxyribonuclease, Sulfatase, Pullulanase, Phosphatase, alpha-Galactosidase, Dextranase, beta-Glucanase-; Lyasen wie Tryptophanase, Tyrosindecarboxylase, Oxinitrilase, Phenylalanindecarboxylase, Pyruvatdecarboxylase, Fumarase, Enolase, Aspartase, Aminolaevulindehydratase, Carboanhydratase; Isomerasen wie Aminosäureracemase, Triosephosphatisomerase; Ligasen wie Glutathionsynthetase.

Aufgrund der Neigung des zyklisch gebundenen basischen Stickstoffs zur Komplexbildung mit Übergangsmetallen sind die erfindungsgemäß erhältlichen Polymeren zur Bindung von Übergangsmetallionen, z. B. Cu, Zn, Fe, Co, Ni, Ru, Rh, Pd, Pt, in verschiedenen Oxidationsstufen befähigt. Diese Komplexe können als Katalysatoren bei verschiedenen Reaktionen Verwendung finden. Beispielsweise wird in DE-OS 24 37 133 die Carboxylierung von Alkoholen in Gegenwart von Polyvinylpyridin-Kupfer-Komplexen beschrieben. Die Verwendung von Pyridin-Palladium-Komplexen als Katalysatoren bei der Herstellung von Isocyanaten ist z. B. aus DE-AS 24 16 683 bekannt. Des weiteren wird in US 3 652 676 als Hydroformylierungskatalysator ein Polyvinylpyridin-Übergangsmetallkomplex angegeben.

Weitere Einsatzgebiete für unlösliche Polymerisate mit basischem Stickstoff sind aus der Literatur bekannt. So weiß man, daß Polymerisate, die Imidazolringe enthalten, die Hydrolyse von z. B. Carbonsäureestern stark beschleunigen (R.L. Letsinger et al., J. Amer. Chem. Soc. 84 (1962) 3122). Polymere mit Pyridinringen sind zur Beschleunigung von Acylierungsreaktionen gut geeignet, wobei sie gleichzeitig zur Säurebindung dienen. Außerdem können Addukte von Polyvinylpyridin mit Brom für Bromierungen, solche mit Chromsäure für Oxidationen eingesetzt werden (J.M. Frechet et al., J. Macromol. Sci. Chem. 11 (1977), 507; J.M. Frechet et al., J. Org. Chem. 43 (1978), 2618).

Die in den Beispielen genannten Teile und Prozente beziehen sich auf das Gewicht.

## Beispiel 1

In einem Rührgefäß mit Rückflußkühler wurden 100 Teile frisch destilliertes 2-Methyl-1-vinylimidazol und 2 Teile N,N'-Divinylethylenharnstoff bei einem Druck von 310 mbar auf 150° C zum Sieden erhitzt. Nach 2,5 Stunden waren in der ursprünglich klaren Flüssigkeit kleine Polymerisatteilchen erkennbar, die langsam zunahmen. Nach 10 Stunden Rühren bestand der Ansatz aus einem trockenen Pulver. Nach Aufnehmen mit 1000 Teilen Wasser, Absaugen auf einer Nutsche, Nachwaschen mit 500 Teilen Wasser und Trocknen in einem Umluftschrank bei 50° C wurden 92 Teile eines leicht bräunlichen Polymerisats erhalten.

## Beispiel 2

Wie bei Beispiel 1 wurden 75 Teile N-Vinylimidazol, 25 Teile N-Vinylpyrrolidon und 3 Teile N,N'-Divinylethylenharnstoff bei 180° C und 250 mbar unter Rühren zum Sieden erhitzt. Nach ca. 1 Stunde waren die ersten Polymerisatteilchen erkennbar, die schnell wuchsen. Nach 2 Stunden Rühren bestand der Ansatz aus einem trockenen Pulver. Nach Waschen und Trocknen wurden 94 Teile eines leicht gelben Polymeren erhalten.

## Beispiel 3

In einem Rührgefäß mit Rückflußkühler wurden 150 Teile frisch destilliertes N-Vinylimidazol, 3 Teile N,N'-Divinylethylenharnstoff und 50 Teile Wasser zum Sieden auf ca. 100° C erhitzt. Nach ca. 3 Stunden entstanden die ersten Polymerisatteilchen, nach 3,5 Stunden bestand der Ansatz aus einem dicken Polymerisatbrei. Nun wurde mit 500 Teilen Wasser verdünnt, wonach die Polymerisatteilchen weiter zunahmen. Nach insgesamt etwa 7 Stunden war die Reaktion beendet. Nun wurde auf einem Filter abgesaugt, das Polymere mit Wasser gewaschen und bei 50° C im Vakuumschrank getrocknet. Das Polymere lag in Form von fast weißen, unregelmäßig geformten Agglomerat-Teilchen von 0,5 bis 2 mm Durchmesser vor. Die Ausbeute betrug 92 %.

## Beispiel 4

In einer Rührapparatur wurden 60 Teile N-Vinylpyrrolidon, 1,2 Teile N,N'-Divinylethylenharnstoff, 540 Teile destilliertes Wasser und 6,65 ml 0,1 n Natronlauge zum Sieden erhitzt. Nach 1/4 Stunde fielen unlösliche Polymerisatteilchen aus der Lösung aus. Nun wurde innerhalb 1,5 Stunden eine Mischung von 540 Teilen frisch destilliertem N-Vinylimidazol und 10,8 Teilen N,N'-Divinylethylenharnstoff zudosiert, wobei die Polymerisatteilchen sehr schnell zunahmen. Um die Suspension rührbar zu halten, wurde nach 1 Stunde, 2 Stunden und 3 Stunden mit jeweils 200 Teilen destilliertem Wasser verdünnt. Nach insgesamt 5 Stunden bei 100° C war die Polymerisation beendet. Das Copolymere lag in Form eines feuchten Pulvers vor. Nach Aufnehmen in 2000 Teilen Wasser wurde das Copolymere abzentrifugiert, mit 2000 Teilen Wasser nachgewaschen und bei 50° C im Vakuumtrockenschrank getrocknet. Das Polymere lag in Form von fast weißen, unregelmäßig geformten Aggregaten von 0,1 bis 3 mm Durchmesser vor. Die Ausbeute betrug 90 %.

15 Teile des nach Beispiel 4 erhaltenen Polymerisats wurden mit 25 Teilen Methyliodid in 200 Teilen Ethanol 5 Stunden auf 60° C erhitzt. Danach wurde abgesaugt, mit Ethanol gewaschen und bei 50° C im Vakuum getrocknet. Nach Überführung in die Chloridform wies das quaternierte Polymere eine Anionenaustauschkapazität von 5,0 mVal/g auf.

**Beispiel 5**

In einer Rührapparatur wurden 60 Teile 4-Vinylpyridin, 1,2 Teile N,N'-Divinylethylenharnstoff, 540 Teile destilliertes Wasser und 6,8 Teile 0,1 n Natronlauge auf 80°C erwärmt. Über das Gemisch wurde während der gesamten Reaktionszeit ein schwacher Stickstoffstrom geleitet. Nach 8 Stunden lag das Polymerisat in Form einer dicken Suspension vor. Das Polymere wurde auf einer Nutsche abgesaugt umd mit 2000 Teilen Wasser gewaschen. Nach dem Trocknen bei 50°C im Vakuumtrockenschrank erhielt man ein fast weißes, körniges Produkt in einer Ausbeute von 90 %.

**Beispiel 6**

Die Polymerisation wurde wie in Beispiel 4 durchgeführt, jedoch wurde als Monomeres 2-Methyl-1-vinylimidazol und als Vernetzer N,N'-Divinylpropylenharnstoff verwendet. Die Ausbeute betrug 96 %. Durch Quaternierung eines Teils des Polymerisates analog Beispiel 4 wurde eine Anionen-Austauschkapazität von 5,1 mVal/g erzielt.

100 mg des nicht quaternierten Polymerisates wurden in 100 ml einer 0,01 %igen Tanninlösung gerührt. Nach 10 Minuten wurde im Überstand die Tanninkonzentration photometrisch bestimmt. Zu diesem Zeitpunkt waren bereits 20 % des Tannins am Polymeren adsorbiert. Nach 40 Minuten waren es 70 %.

7 g des nicht quaternierten Polymerisates wurden 2 Tage in 70 ml einer 1 %igen Lösung von Invertase gerührt. Nach dieser Zeit waren 90 % des Enzyms gebunden. Danach wurde das Polymerisat in eine Säule überführt und bei 30°C mit einer Fließgeschwindigkeit von 100 ml/h eine 64 %ige Saccharose-Lösung darübergeleitet. Der Hydrolysegrad der Saccharose betrug nach 20 Tagen 81 %, nach 40 Tagen 80 %, nach 60 Tagen 80,5 %.

**Beispiel 7**

In einer Rührapparatur wurden 15 Teile Vinylpyrrolidon, 0,45 Teile Divinylethylenharnstoff, 135 Teile Wasser und 1,65 Teile 0,1 n Natronlauge im Stickstoffstrom auf 85°C erhitzt. Nun wurden 0,03 Teile Natriumdithionit, gelöst in 10 Teilen Wasser, zugesetzt, und nach ca. 40 Minuten, als deutlich unlösliche Polymerisatteilchen erkennbar waren, wurde eine Mischung von 120 Teilen 1-Vinyl-2-methylimidazol, 15 Teilen Methylacrylat, 4,05 Teilen Methylenbisacrylamid und 500 Teilen Wasser innerhalb 20 Minuten zudosiert. Anschließend wurde noch 1 Stunde bei 85°C erhitzt. Nach Abkühlen wurde der dicke Polymerisatbrei abfiltriert, mit Wasser gewaschen und bei 50°C im Vakuumtrockenschrank getrocknet. Das Polymere lag in Form von weißen, unregelmäßig geformten Aggregaten von etwa 0,1 bis 5 mm Durchmesser vor. Die Ausbeute betrug 93,5 %.

**Beislpiel 8**

Die Polymerisation wurde wie bei Beispiel 7 durchgeführt, jedoch nachdem in der Vorlage Polymerisatteilchen gebildet waren, wurde eine Mischung von 1,05 Teilen 1-Vinyl-2-methylimidazol, 30 Teilen Vinylacetat, 4,05 Teilen N,N'-Bisacryloylethylendiamin und 500 Teilen Wasser zudosiert. Die Ausbeute betrug 82,5 %.

**Vergleichsbeispiel:**

Mit Radikalbildner hergestelltes vernetztes Polyvinylimidazol

100 Teile Vinylimidazol wurden mit 2 Teilen N,N'-Methylenbisacrylamid und 2 Teilen Azodiisobuttersäurenitril in 500 Teilen Wasser gelöst und 4 h auf 80°C erwärmt. Das erhaltene steife Gel wurde im Vakuum bei 50°C getrocknet. Man erhielt ein glasartiges Polymerisat in nahezu quantitativer Ausbeute. Zur Bestimmung des Quellverhaltens wurde das Polymere zerkleinert, und eine Siebfraktion von 250 bis 500 μm wurde in Wasser 2 h gequollen. Das gequollene Polymere wurde auf einer Nutsche scharf abgesaugt, feucht abgewogen, im Vakuum bei 80°C getrocknet und wieder gewogen. Es ergab sich eine Wasseraufnahme von 12,9 g/g Polymer.

Ein nach Beispiel 4 hergestelltes Polymerisat ergab bei gleichem Vorgehen eine Wasseraufnahme von 1,7 g/g Polymer.

**Patentansprüche**

1. Verfahren zur Herstellung von unlöslichen in Wasser nur wenig quellbaren, körnigen Polymerisaten eines gesättigten oder aromatisch ungesättigten Heterocyclus mit einer Vinylgruppe und mindestens einen besischen tertiären Ring-Stickstoffatom mit einem pKa > 4 und dessen Copolymerisaten mit bis zu 30 Gew.% copolymerisierbarer Monomerer durch Polymerisieren der Mononeren, gekennzeichnet durch die Kombination folgender Maßnahmen

a) Einsatz von 0,1 bis 10 Gew.%, bezogen auf die Gesamtmonomermenge, eines Vernetzers,

b) Ausschluß von Sauerstoff,

c) Ausschluß von Polymerisationsinitiatoren.

2. Verfahren nach Anspruch 1, _dadurch gekennzeichnet_, daß es in Gegenwart von Wasser bei 20 bis 150° C durchgefürt wird.

3. Verfahren nach Anspruch 1, _dadurch gekennzeichnet_, daß es ohne Lösungsmittel bei 100 bis 200° C durchgeführt wird.

4. Verfahren nach Anspruch 1 bis 3, _dadurch gekennzeichnet_, daß es in Gegenwart eines Reduktionsmittels durchgeführt wird.

5. Verfahren nach Anspruch 1 bis 4, _dadurch gekennzeichnet_, daß als basischer Vinylheterocyclus 1-Vinylimidazol oder 2-Methyl-1-vinylimidazol eingesetzt wird.

6. Verfahren nach Anspruch 1 bis 5, _dadurch gekennzeichnet_, daß als basischer Vinylheterocyclus 2-Vinylpyridin-4-Vinylpyridin oder 2-Methyl-5-vinylpyridin eingesetzt wird.

7. Verfahren nach Anspruch 1 bis 6, _dadurch gekennzeichnet_, daß als Comonomeres ein N-Vinyllactam eingesetzt wird.

8. Verfahren nach Anspruch 1 bis 7, _dadurch gekennzeichnet_, daß als Comonomeres N-Vinylpyrrolidon eingesetzt wird.

9. Verfahren nach Anspruch 1 bis 8, _dadurch gekennzeichnet_, daß als Vernetzer N,N'-Divinylethylenharnstoff verwendet wird.

10. Verfahren nach Anspruch 1 bis 9, _dadurch gekennzeichnet_, daß die basischen Stickstoffatome der Vinylheterocyclen nach der Polymerisation mit einem üblichen Alkylierungsmittel vollständig oder teilweise quaterniert werden.

11. Verwendung der nach einem der Ansprüche 1 bis 10 hergestellten Polymeren als Ionenaustauscher.

12. Verwendung der nach einen der Ansprüche 1 bis 10 hergestellten Polymeren als Adsorberharze.

13. Verwendung der nach einem der Ansprüche 1 bis 10 hergestellten Polymeren zur Adsorption von Proteinen aus wäßriger Lösung.

14. Verwendung der nach einem der Ansprüche 1 bis 10 hergestellten Polymeren zur adsorptiven Immobilisierung von Enzymen.

15. Verwendung der nach einem der Ansprüche 1 bis 10 hergestellten Polymeren nach Adsorption eines Enzyms als heterogener Katalysator zur Durchführung der entsprechenden Enzymreaktion.

16. Verwendung der nach einem der Ansprüche 1 bis 10 hergestellten Polymeren zur Bindung von Übergangsmetallionen.

17. Verwendung der nach einem der Ansprüche 1 bis 10 hergestellten Polymeren als Solvolysekatalysatoren.

18. Verwendung der nach einem der Ansprüche 1 bis 10 hergestellten Polymeren als Acylierungskatalysatoren.

## Claims

1. A process for the preparation of insoluble, only slightly water-swellable, granular polymers of a saturated or aromatically unsaturated heterocyclic compound containing one vinyl group and one or more basic tertiary ring nitrogen atoms having a pKa greater than 4, and of their copolymers with up to 30 % by weight of copolymerizable monomers, by polymerizing the monomers, wherein the following measures are combined:.

a) 0.1 to 10 % by weight, based on the total amount of monomer, of a crosslinking agent is employed,

b) oxygen is excluded, and

c) polymerization initiators are excluded.

2. A process as claimed in claim 1, wherein the polymerization is carried out in the presence of water at from 20 to 150° C.

3. A process as claimed in claim 1, wherein the polymerization is carried out in the absence of a solvent at from 100 to 200° C.

4. A process as claimed in claims 1 to 3, wherein the polymerization is carried out in the presence of a reducing agent.

5. A process as claimed in claims 1 to 4, wherein the basic vinyl-heterocyclic compound employed is 1-vinylimidazole or 2-methyl-1-vinylimidazole.

6. A process as claimed in claims 1 to 5, wherein the basic vinyl-heterocyclic compound employed is 2-vinylpyridine, 4-vinylpyridine or 2-methyl-5-vinylpyridine.

7. A process as claimed in claims 1 to 6, wherein the comonomer employed is an N-vinyllactam.

8. A process as claimed in claims 1 to 7, wherein the comonomer employed is N-vinylpyrrolidone.

9. A process as claimed in claims 1 to 8, wherein the crosslinking agent employed is N,N'-divinylethylene-urea.

10. A process as claimed in claims 1 to 9, wherein, after the polymerization, the basic nitrogen atoms of the vinylheterocyclic compound are completely or partially quaternized with a conventional alkylating agent.

11. The use of a polymer prepared as claimed in any of claims 1 to 10 as an ion exchanger.

12. The use of a polymer prepared as claimed in any of claims 1 to 10 as an adsorbent resin.

13. The use of a polymer prepared as claimed in any of claims 1 to 10 for adsorbing proteins from aqueous solutions.

14. The use of a polymer prepared as claimed in any of claims 1 to 10 for the adsorbtive immobilization of enzymes.

15. The use of a polymer prepared as claimed in any of claims 1 to 10, after adsorption of an enzyme, as a heterogeneous catalyst for carrying out the corresponding enzymatic reaction.

16. The use of a polymer prepared as claimed in any of claims 1 to 10 for binding transition metal ions.

17. The use of a polymer prepared as claimed in any of claims 1 to 10 as a solvolysis catalyst.

18. The use of a polymer prepared as claimed in any of claims 1 to 10 as an acylation catalyst.

**Revendications**

1. Procédé de préparation de polymères granulés insolubles, gonflant peu dans l'eau, d'un composé hétérocyclique saturé ou à insaturation aromatique, comprenant un groupe vinylique et au moine un atome d'azote tertiaire basique dans le noyau avec un pKa supérieur à 4, et de copolymères de celui-ci avec jusqu'à 30 % en poids de monomères copolymérisés, par polymérisation des monomères, caractérisé par la combinaison des mesures ci-après:

a) l'utilisation d'un agent de réticulation à raison de 0,1 à 10 % du poids total des monomères;

b) le travail à l'abri de l'oxygène et

c) l'absence de tout amorceur de la polymérisation.

2. Procédé suivant la revendication 1, caractérisé en ce qu'il est réalisé en présence d'eau et entre 20 et 150°C

3. Procédé suivant la revendication 1, caractérisé en ce qu'il est réalisé entre 100 et 200°C en l'absence d'un solvant.

4. Procédé suivant l'une des revendications 1 à 3, caractérisé en ce qu'il est réalisé en présence d'un agent réducteur.

5. Procédé suivant l'une des revendications 1 à 4, caractérisé en ce que le composé vinyl-hétérocyclique basique est le vinyl-1 imidazole ou le méthyl-2 vinyl-1 imidazole.

6. Procédé suivant l'une des revendications 1 à 5, caractérisé en ce que le composé vinyl-hétérocyclique est la vinyl-2 pyridine, la vinyl-4 pyridine ou la méthyl-2 vinyl-5 pyridine.

7. Procédé suivant l'une des revendications 7 à 6, caractérisé en ce que le comonomère est une N-vinyllactame.

8. Procédé suivant l'une des revendications 1 à 7, caractérisé en ce que le comonomère est la N-vinylpyrrolidone.

9. Procédé suivant l'une des revendications 1 à 8, caractérisé en ce que l'agent de réticulation est la N,N'-divinyl-éthylène-urée.

10. Procédé suivant l'une des revendications 1 à 9, caractérisé en ce que, après la polymérisation, les atomes d'azote basiques des composés vinyl-hétérocycliques sont complètement ou partiellement quaternisés à l'aide d'un agent d'alkylation usuel.

11. Utilisation des polymères préparés selon l'une des revendications 1 à 10 comme résines échangeuses d'ions.

12. Utilisation des polymères préparés selon l'une des revendications 1 à 10 comme résines adsorbantes.

13. Utilisation des polymères préparés selon l'une des revendications 1 à 10 pour l'adsorption de protéines en solution aqueuse.

14. Utilisation des polymères préparés selon l'une des revendications 1 à 10 pour l'immobilisation d'enzymes par adsorption.

15. Utilisation des polymères préparés selon l'une des revendications 1 à 10 après l'adsorption d'une enzyme en tant que catalyseur hétérogène pour la réalisation de la réaction enzymatique correspondante.

16. Utilisation des polymères préparés selon l'une des revendications 1 à 10 pour la fixasion d'ions de métaux de transition.

17. Utilisation des polymères préparés selon l'une des revendications 1 à 10 comme catalyseurs de solvolyse.

18. Utilisation des polymères préparés selon l'une des revendications 1 à 10 comme catalyseurs d'acylation.